# EUROPEAN PATENT SPECIFICATION

(11) **EP 1 689 799 B1**
(45) Date of publication and mention of the grant of the patent: **20.01.2016**
(21) Application number: 04819689.3
(22) Date of filing: 24.11.2004
(51) Int. Cl.: C08G 18/76, C08K 5/134, C07C 69/732

(54) **STABILIZATION OF POLYETHER POLYOLS, POLYESTER POLYOLS AND POLYURETHANES**
STABILISIERUNG VON POLYETHERPOLYOLEN, POLYESTERPOLYOLEN UND POLYURETHANEN
STABILISATION DE POLYOLS DE POLYETHER, DE POLYOLS DE POLYESTER ET DE POLYURETHANNES

(30) Priority: 04.12.2003 EP 03104540
(43) Date of publication of application: 16.08.2006
(73) Proprietor: BASF SE, 67056 Ludwigshafen (DE)
(72) Inventor: MÄDER, Dietmar, 79104 Freiburg (DE); SCHRINNER, Kerstin, Shanghai 200031 (CN); XANTHOPOULOS, Pascal, Singapore 599140 (SG)
(74) Representative: Zumstein, Angela
(86) International application number: PCT/EP2004/053068
(87) International publication number: WO 2005/054328

(56) References cited:
- WO-A2-02/00750
- US-A- 3 285 855
- US-A- 3 330 859
- US-A- 4 228 297
- US-A- 5 354 486
- US-A- 5 523 007
- US-A- 5 739 341
- US-A- 5 821 206
- US-B1- 6 303 052
- CLAUSS M ET AL: "ANTIOXIDANT SYSTEMS FOR STABILIZATION OF FLEXIBLE POLYURETHANE SLABSTOCK FOAMS" JOURNAL OF CELLULAR PLASTICS, TECHNOMIC PUBLISHING CO.INC. WESTPORT CONN, US, vol. 33, no. 5, 1997, pages 457-476, XP009008914 ISSN: 0021-955X

## Description

The present invention relates to compositions comprising a polyether polyol, a polyester polyol or a polyurethane susceptible to oxidative, thermal or light-induced degradation, as stabilizer a specific group of liquid phenolic antioxidants and as processing stabilizers benzofuranones, phosphites, phosphonites or phenothiazines.

The use of phenolic antioxidants as stabilizers for polyether polyols, polyester polyols or polyurethanes is known, for example, from H. Zweifel; Plastic Additives Handbook, 5th Edition, Hanser Publishers, Munich, pages 88 - 109 (2001).

The known stabilizers do not satisfy in every respect the high requirements which a stabilizer is required to meet, especially with regard to shelf life, water absorption, sensitivity to hydrolysis, in-process stabilization, color properties, volatility, migration behavior, compatibility and improvement in protection against light. Additionally, there is a strong demand from the automotive industry to significantly reduce of the amount of volatile organic compounds (VOC) and especially gaseous emissions (FOG). The gaseous emissions are also often related to the "fogging" phenomenon, where evaporated volatile materials may condensate in automobile windscreens leading to deposits on the window. In addition, end-users of bedding, furniture and carpet backing foam are also putting pressure on the manufacturers of flexible slabstock foam. Co-additives such as catalysts, surfactants, flame retardants, antioxidants contribute to emissions of the polyurethane foams. The main sources of VOC are additive like silicone surfactants and amine catalysts. The state-of-the art for the stabilization of flexible slabstock is based on combinations of hindered phenols and second-dary aromatic amines. Especially liquid phenolic antioxidants contribute to gaseous emissions (FOG).

The present invention relates to a specific group of liquid phenolic antioxidants with extremely low contribution to fogging.

The present invention therefore provides compositions comprising
(a) a polyether polyol, a polyester polyol or a polyurethane susceptible to oxidative, thermal or light-induced degradation; and
(b) at least a liquid compound of the formula I 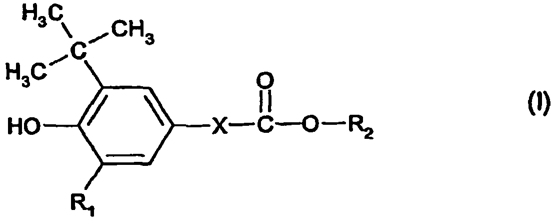 wherein
   R1 is C₁-C₄alkyl,
   R₂ is a branched C₁₂-C₂₅alkyl, and
   X is C₁-C₈alkylene or C₁-C₄alkyl substituted C₂-C₈alkylene; and
c) as processing stabilizers benzofuranones, phosphites, phosphonites or phenothiazines.

Alkyl having up to 4 carbon atoms is a branched or unbranched radical, for example methyl, ethyl, propyl, isopropyl, n-butyl, sec-butyl, isobutyl or tert-butyl. One of the preferred definitions for R, is methyl or tert-butyl.

Alkyl having between 12 and 25 carbon atoms is a branched radical, for example 2-butyl-1-octanol, 2-butyl-1-nonaol, 2-butyl-1-decanol, 2-butyl-1-undecanol, 2-butyl-1-dodecanol, 2-butyl-1-octadecanol, 2-pentyl-1-octanol, 2-pentyl-1-nonaol, 2-pentyl-1-decanol, 2-pentyl-1-undecanol, 2-pentyl-1-dodecanol, 2-pentyl-1-octadecanol, 2-hexyl-1-octanol, 2-hexyl-1-nonaol, 2-hexyl-1-decanol, 2-hexyl-1-undecanol, 2-hexyl-1-dodecanol, 2-hexyl-1-octadecanol, 2-heptyl-1-octanol, 2-heptyl-1-nonaol, 2-heptyl-1-decanol, 2-heptyl-1-undecanol, 2-heptyl-1-dodecanol, 2-heptyl-1-octadecanol, 2-octyl-1-octanol, 2-octyl-1-nonaol, 2-octyl-1-decanol, 2-octyl-1-undecanol, 2-octyl-1-dodecanol or 2-octyl-1-octadecanol. A preferred definition for R₂ is 2-hexyl-1-decanol and 2-octyl-1-dodecanol.

C₁-C₈Alkylene is a branched or unbranched radical, for example methylene, ethylene, propylene, trimethylene, tetramethylene, pentamethylene, hexamethylene, heptamethylene or octamethylene. A preferred definition for X is C₁-C₄alkylene, especially ethylene.

C₁-C₄Alkyl substituted C₁-C8alkylene is a branched or unbranched radical, for example ethylidene, 1-methylethylene, 2-methylethylene, 1-methylpropylene or 2-methylpropylene. A preferred definition for X is 1-methylethylene or 2-methylethylene.

Preferred compounds of the formula I are those wherein
R₁ is methyl or tert-butyl,
R₂ is a branched C₁₄-C₂₅alkyl, and
X is C₁-C₄alkylene or a methyl substituted C₂-C₄alkylene.

Further preferred compounds of the formula I are those wherein X is ethylene.

Of special interest are compounds of the formula I wherein R₂ is a branched C₁₅-C₂₂alkyl.

Preferably, R₂ is 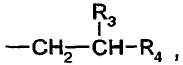 wherein
R₃ is C₃-C₁₈alkyl, and
R₄ is C₅-C₂₀alkyl; with the proviso that the sum of carbon atoms of R₃ and R₄ is from 10 to 23.

Also of interest are compounds of the formula I wherein
R₁ is methyl or tert-butyl,
R₂ is a branched C₁₆-C₂₀alkyl, and
X is ethylene.

The compounds of the formula I can be prepared in per se known manner, for example by esterification of an carboxylic acid with an alcohol.

The compounds of the formula I are suitable as stabilizers for polyether polyols, polyester polyols or polyurethanes against oxidative, thermal or light-induced degradation and as reducers of fogging contribution of the polymers.

The compounds of the formula I are likewise used for polyurethane production, especially for preparing flexible polyurethane foams. In this context the novel compositions and the products produced therefrom are effectively protected against degradation. In particular, scorching during foam production is avoided. Preferably, phosphites such as for example diphenyl isodecyl phosphite (DPDP) or phenyl diisodecyl phosphite (PDDP) are post added as antioxidants or antiscorch systems to the base stabilized polyether polyols at the mixing head prior to the foaming in relative high concentrations (up to 1.5 % by weight based on the polyether polyol).

The polyurethanes are obtained, for example, by reacting polyethers, polyesters and polybutadienes which contain terminal hydroxyl groups with aliphatic or aromatic polyisocyanates.

Polyethers and polyesters having terminal hydroxyl groups are known and are prepared, for example, by polymerizing epoxides such as ethylene oxide, propylene oxide, butylene oxide, tetrahydrofuran, styrene oxide or epichlorohydrin with themselves, for example in the presence of BF₃, or by addition reaction of these epoxides, alone or as a mixture or in successsion, with starting components containing reactive hydrogen atoms, such as water, alcohols, ammonia or amines, for example ethylene glycol, propylene 1,3- and 1,2-glycol, trimethylolpropane, 4,4'-dihydroxydiphenylpropane, aniline, ethanolamine or ethylenediamine. Sucrose polyethers are also suitable in accordance with the invention. In many cases preference is given to those polyethers which predominantly (up to 90 % by weight, based on all the OH groups present in the polyether) contain primary OH groups. Furthermore, polyethers modified by vinyl polymers, as are formed, for example, by polymerizing styrene and acrylonitrile in the presence of polyethers, are suitable, as are polybutadienes containing OH groups.

These compounds generally have molecular weights of 40 and are polyhydroxy compounds, especially compounds containing from two to eight hydroxyl groups, especially those of molecular weight from 800 to 10 000, preferably from 1000 to 6000, for example polyethers containing at least 2, generally 2 to 8, but preferably 2 to 4, hydroxyl groups, as are known for the preparation of homogeneous polyurethanes and cellular polyurethanes.

It is of course possible to employ mixtures of the above compounds containing at least two isocyanate-reactive hydrogen atoms, in particular with a molecular weight of 400 - 10 000.

Suitable polyisocyanates are aliphatic, cycloaliphatic, araliphatic, aromatic and heterocyclic polyisocyanates, for example ethylene diisocyanate, 1,4-tetramethylene diisocyanate, 1,6-hexamethylene diisocyanate, 1,12-dodecane diisocyanate, cyclobutane 1,3-diisocyanate, cyclohexane 1,3- and -1,4-diisocyanate and also any desired mixtures of these isomers, 1-isocyanato-3,3,5-trimethyl-5-isocyanatomethylcyclohexane, 2,4- and 2,6-hexahydrotolylene diisocyanate and also any desired mixtures of these isomers, hexahydro-1,3- and/or -1,4-phenylene diisocyanate, perhydro-2,4'- and/or -4,4'-diphenylmethanediisocyanate, 1,3- and 1,4-phenylene diisocyanate, 2,4- and 2,6-tolylene diisocyanate, and also any desired mixtures of these isomers, diphenylmethane 2,4'- and/or -4,4'-diisocyanate, naphthylene 1,5-diisocyanate, triphenylmethane 4,4',4"-triisocyanate, polyphenyl-polymethylene polyisocyanates as are obtained by aniline-formaldehyde condensation followed by phosgenization, m- and p-isocyanatophenylsulfonyl isocyanates, perchlorinated aryl polyisocyanates, polyisocyanates containing carbodiimide groups, polyisocyanates containing allophanate groups, polyisocyanates containing isocyanurate groups, polyisocyanates containing urethane groups, polyisocyanates containing acylated urea groups, polyisocyanates containing biuret groups, polyisocyanates containing ester groups, reaction products of the abovementioned isocyanates with acetals, and polyisocyanates containing polymeric fatty acid radicals.

It is also possible to employ the isocyanate group-containing distillation residues as they are or dissolved in one or more of the abovementioned polyisocyanates, which are obtained in the course of the industrial preparation of isocyanates. It is additionally possible to use any desired mixtures of the abovementioned polyisocyanates.

Particular preference is given in general to the polyisocyanates which are readily obtainable industrially, for example 2,4- and 2,6-tolylene diisocyanate and any desired mixtures of these isomers ("TDI"), polyphenyl-polymethylene-polyisocyanates as prepared by aniline-formaldehyde condensation followed by phosgenization ("crude MDI"), and polyisocyanates containing carbodiimide, urethane, allophanate, isocyanurate, urea or biuret groups ("modified polyisocyanates").

Polyurethane foams are preferably prepared from liquid starting components, either the starting materials to be reacted with one another being mixed together in a one-shot process, or a preadduct containing NCO groups that are formed from a polyol and an excess of polyisocyanate being prepared first and the foamed, typically by reaction with water.

In the preparation of foams, the foaming is often carried out in moulds. In that case, the reaction mixture is placed in a mould. Suitable mould materials are metals, typically aluminium, or plastics, typically epoxy resins. In the mould, the foamable reaction mixture foams up and forms the moulded article. The foam moulding can be carried out such that the moulding has a cellular surface structure or, alternatively, such that the moulding has a dense skin and a cellular core. In this connection, it is possible to place into the mould a sufficient amount of foamable reaction mixture for the foam obtained to fill the mould exactly. It is, however, also possible to place more foamable reaction mixture into the mould than is required to fill the interior of the mould with foam. In the latter case, therefore, the operation is carried out with overcharging.

In the case of foam moulding, known external release agents, typically silicone oils, are often used concomitantly. It is, however, also possible to use so-called internal release agents, optionally in admixture with external release agents. It is also possible to use cold-curing foams. The foams can, of course, alternatively be prepared by block foaming or by the known double conveyor belt process. These processes can be used to prepare flexible, semi-flexible or hard polyurethane foams. The foams find the utilities known for such products, for example as mattresses and upholstery in the furniture and automobile industries, as well as for the manufacture of fittings, such as are used in the automobile industry, and finally as sound-insulating compositions and as compositions for heat-insulation and low-temperature insulation, for example in the construction sector or in the refrigeration industry, or in the textile industry, for example as shoulder pads.

The compounds of the formula I are preferably added to the polymer to be stabilized in an amount of from 0.01 to 10 %, in particular from 0.01 to 5 %, for example from 0.01 to 2 %, based on the weight of the polymer to be stabilized.

In addition to components (a) and (b) the novel compositions may comprise further costabilizers (additives) such as, for example, the following:
1. Antioxidants
   1.1. Alkylated monophenols, for example 2,6-di-tert-butyl-4-methylphenol, 2-tert-butyl-4,6-dimethylphenol, 2,6-di-tert-butyl-4-ethylphenol, 2,6-di-tert-butyl-4-n-butylphenol, 2,6-di-tert-butyl-4-isobutylphenol, 2,6-dicyclopentyl-4-methylphenol, 2-(α-methylcyclohexyl)-4,6-dimethyl-phenol, 2,6-dioctadecyl-4-methylphenol, 2,4,6-tricyclohexylphenol, 2,6-di-tert-butyl-4-meth-oxymethylphenol, nonylphenols which are linear or branched in the side chains, for example 2,6-di-nonyl-4-methylphenol, 2,4-dimethyl-6-(1'-methylundec-1'-yl)phenol, 2,4-dimethyl-6-(1'-methylheptadec-1'-yl)phenol, 2,4-dimethyl-6-(1'-methyltridec-1-yl)phenol and mixtures thereof.
   1.2. Alkylthiomethyphenols, for example 2,4-dioctylthiomethyl-6-tert-butylphenol, 2,4-dioctylthiomethyl-6-methylphenol, 2,4-dioctylthiomethyl-6-ethylphenol, 2,6-di-dodecylthiomethyl-4-nonylphenol.
   1.3. Hydroquinones and alkylated hydroquinones, for example 2,6-di-tert-butyl-4-methoxyphenol, 2,5-di-tert-butylhydroquinone, 2,5-di-tert-amylhydroquinone, 2,6-diphenyl-4-octadecyloxyphenol, 2,6-di-tert-butylhydroquinone, 2,5-di-tert-butyl-4-hydroxyanisole, 3,5-di-tert-butyl-4-hydroxyanisole, 3,5-di-tert-butyl-4-hydroxyphenyl stearate, bis(3,5-di-tert-butyl-4-hydroxyphenyl) adipate.
   1.4. Tocopherols, for example α-tocopherol, β-tocopherol, γ-tocopherol, δ-tocopherol and mixtures thereof (vitamin E).
   1.5. Hydroxylated thiodiphenyl ethers, for example 2,2'-thiobis(6-tert-butyl-4-methylphenol), 2,2'-thiobis(4-octylphenol), 4,4'-thiobis(6-tert-butyl-3-methylphenol), 4,4'-thiobis(6-tert-butyl-2-methyl phenol), 4,4'-thiobis(3,6-di-sec-amylphenol), 4,4'-bis(2,6-dimethyl-4-hydroxyphenyl)disulfide.
   1.6. Alkylidenebisphenols, for example 2,2'-methylenebis(6-tert-butyl-4-methylphenol), 2,2'-methylenebis(6-tert-butyl-4-ethylphenol), 2,2'-methylenebis[4-methyl-6-(α-methylcyclohexyl)-phenol], 2,2'-methylenebis(4-methyl-6-cyclohexylphenol), 2,2'-methylenebis(6-nonyl-4-methylphenol), 2,2'-methylenebis(4,6-di-tert-butylphenol), 2,2'-ethylidenebis(4,6-di-tert-butyl-phenol), 2,2'-ethylidenebis(6-tert-butyl-4-isobutylphenol), 2,2'-methylenebis[6-(α-methylbenzyl)-4-nonylphenol], 2,2'-methylenebis[6-(α,α-dimethylbenzyl)-4-nonylphenol], 4,4'-methylenebis(2,6-di-tert-butylphenol), 4,4'-methylenebis(6-tert-butyl-2-methylphenol), 1,1-bis(5-tert-butyl-4-hydroxy-2-methylphenyl)butane, 2,6-bis(3-tert-butyl-5-methyl-2-hydroxybenzyl)-4-methylphenol, 1,1,3-tris(5-tert-butyl-4-hydroxy-2-methylphenyl)butane, 1,1-bis(5-tert-butyl-4-hydroxy-2-methylphenyl)-3-n-dodecylmercaptobutane, ethylene glycol bis[3,3-bis(3'-tert-butyl-4'-hydroxyphenyl)butyrate], bis(3-tert-butyl-4-hydroxy-5-methyl-phenyl)dicyclopentadiene, bis[2-(3'-tert-butyl-2'-hydroxy-5'-methylbenzyl)-6-tert-butyl-4-methylphenyl]terephthalate, 1,1-bis-(3,5-dimethyl-2-hydroxyphenyl)butane, 2,2-bis(3,5-di-tert-butyl-4-hydroxyphenyl)propane, 2,2-bis-(5-tert-butyl-4-hydroxy2-methylphenyl)-4-n-dodecylmercaptobutane, 1,1,5,5-tetra(5-tert-butyl-4-hydroxy-2-methylphenyl)pentane.
   1.7. O-, N- and S-benzyl compounds, for example 3,5,3',5'-tetra-tert-butyl-4,4'-dihydroxydibenzyl ether, octadecyl-4-hydroxy-3,5-dimethylbenzylmercaptoacetate, tridecyl-4-hydroxy-3,5-di-tert-butylbenzylmercaptoacetate, tris(3,5-di-tert-butyl-4-hydroxybenzyl)amine, bis(4-tert-butyl-3-hydroxy-2,6-dimethylbenzyl)dithioterephthalate, bis(3,5-di-tert-butyt-4-hydroxybenzyl)sulfide, isooctyl-3,5-di-tert-butyl-4-hydroxybenzylmercaptoacetate.
   1.8. Hydroxybenzylated malonates, for example dioctadecyl-2,2-bis(3,5-di-tert-butyl-2-hydroxybenzyl)malonate, di-octadecyl-2-(3-tert-butyl-4-hydroxy-5-methylbenzyl)malonate, didodecylmercaptoethyl-2,2-bis(3,5-di-tert-butyl-4-hydroxybenzyl)malonate, bis[4-(1,1,3,3-tetramethylbutyl)phenyl]-2,2-bis(3,5-di-tert-butyl-4-hydroxybenzyl)malonate.
   1.9. Aromatic hydroxybenzyl compounds, for example 1,3,5-tris(3,5-di-tert-butyl-4-hydroxybenzyl)-2,4,6-trimethylbenzene, 1,4-bis(3,5-di-tert-butyl-4-hydroxybenzyl)-2,3,5,6-tetramethylbenzene, 2,4,6-tris(3,5-di-tert-butyl-4-hydroxybenzyl)phenol.
   1.10. Triazine compounds, for example 2,4-bis(octylmercapto)-6-(3,5-di-tert-butyl-4-hydroxyanilino)-1,3,5-triazine, 2-octylmercapto-4,6-bis(3,5-di-tert-butyl-4-hydroxyanilino)-1,3,5-triazine, 2-octylmercapto-4,6-bis(3,5-di-tert-butyl-4-hydroxyphenoxy)-1,3,5-triazine, 2,4,6-tris-(3,5-di-tert-butyl-4-hydroxyphenoxy)-1,2,3-triazine, 1,3,5-tris(3,5-di-tert-butyl-4-hydroxybenzyl)isocyanurate, 1,3,5-tris(4-tert-butyl-3-hydroxy-2,6-dimethylbenzyl)isocyanurate, 2,4,6-tris(3,5-di-tert-butyl-4-hydroxyphenylethyl)-1,3,5-triazine, 1,3,5-tris(3,5-di-tert-butyl-4-hydroxyphenylpropionyl)-hexahydro-1,3,5-triazine, 1,3,5-tris(3,5-dicyclohexyl-4-hydroxybenzyl)isocyanurate.
   1.11. Benzylphosphonates, for example dimethyl-2,5-di-tert-butyl-4-hydroxybenzylphosphonate, diethyl-3,5-di-tert-butyl-4-hydroxybenzylphosphonate, dioctadecyl3,5-di-tert-butyl-4-hydroxybenzylphosphonate, dioctadecyl-5-tert-butyl-4-hydroxy-3-methylbenzylphosphonate, the calcium salt of the monoethyl ester of 3,5-di-tert-butyl-4-hydroxybenzylphosphonic acid.
   1.12. Acylaminophenols, for example 4-hydroxylauranilide, 4-hydroxystearanilide, octyl N-(3,5-di-tert-butyl-4-hydroxyphenyl)carbamate.
   1.13. Esters of β-(3,5-di-tert-butyl-4-hydroxyphenyl)propionic acid with mono- or polyhydric alcohols, e.g. with methanol, ethanol, n-octanol, i-octanol, octadecanol, 1,6-hexanediol, 1,9-nonanediol, ethylene glycol, 1,2-propanediol, neopentyl glycol, thiodiethylene glycol, diethylene glycol, triethylene glycol, pentaerythritol, tris(hydroxyethyl)isocyanurate, N,N'-bis(hydroxyethyl)oxamide, 3-thiaundecanol, 3-thiapentadecanol, trimethylhexanediol, trimethylolpropane, 4-hydroxymethyl-1-phospha-2,6,7-trioxabicyclo[2.2.2]octane.
   1.14. Esters of β-(5-tert-butyl-4-hydroxy-3-methylphenyl)propionic acid with mono- or polyhydric alcohols, e.g. with methanol, ethanol, n-octanol, i-octanol, octadecanol, 1,6-hexanediol, 1,9-nonanediol, ethylene glycol, 1,2-propanediol, neopentyl glycol, thiodiethylene glycol, diethylene glycol, triethylene glycol, pentaerythritol, tris(hydroxyethyl)isocyanurate, N,N'-bis(hydroxyethyl)oxamide, 3-thiaundecanol, 3-thiapentadecanol, trimethylhexanediol, trimethylolpropane, 4-hydroxymethyl-1-phospha-2,6,7-trioxabicyclo[2.2.2]octane; 3,9-bis[2-{3-(3-tert-butyl-4-hydroxy-5-methylphenyl)propionyloxy}-1,1-dimethylethyl]-2,4,8,10-tetraoxaspiro[5.5]undecane.
   1.15. Esters of β-(3,5-dicyclohexyl-4-hydroxyphenyl)propionic acid with mono- or polyhydric alcohols, e.g. with methanol, ethanol, octanol, octadecanol, 1,6-hexanediol, 1,9-nonanediol, ethylene glycol, 1,2-propanediol, neopentyl glycol, thiodiethylene glycol, diethylene glycol, triethylene glycol, pentaerythritol, tris(hydroxyethyl)isocyanurate, N,N'-bis(hydroxyethyl)oxamide, 3-thiaundecanol, 3-thiapentadecanol, trimethylhexanediol, trimethylolpropane, 4-hydroxymethyl-1-phospha-2,6,7-trioxabicyclo[2.2.2]octane.
   1.16. Esters of 3,5-di-tert-butyl-4-hydroxyphenyl acetic acid with mono- or polyhydric alcohols, e.g. with methanol, ethanol, octanol, octadecanol, 1,6-hexanediol, 1,9-nonanediol, ethylene glycol, 1,2-propanediol, neopentyl glycol, thiodiethylene glycol, diethylene glycol, triethylene glycol, pentaerythritol, tris(hydroxyethyl)isocyanurate, N,N'-bis(hydroxyethyl)oxamide, 3-thiaundecanol, 3-thiapentadecanol, trimethylhexanediol, trimethylolpropane, 4-hydroxymethyl-1-phospha-2,6,7-trioxabicyclo[2.2.2]octane.
   1.17. Amides of β-(3,5-di-tert-butyl-4-hydroxyphenyl)propionic acid e.g. N,N'-bis(3,5-di-tert-butyl-4-hydroxyphenylpropionyl)hexamethylenediamide, N,N'-bis(3,5-di-tert-butyl-4-hydroxyphenylpropionyl)trimethylenediamide, N,N'-bis(3,5-di-tert-butyl-4-hydroxyphenylpropionyl)hydrazide, N,N'-bis[2-(3-[3,5-di-tert-butyl-4-hydroxyphenyl]propionyloxy)ethyl]oxamide (Naugard^{®}XL-1, supplied by Uniroyal).
   1.18. Ascorbic acid (vitamin C)
   1.19. Aminic antioxidants, for example N,N'-di-isopropyl-p-phenylenediamine, N,N'-di-sec-butyl-p-phenylenediamine, N,N'-bis(1,4-dimethylpentyl)-p-phenylenediamine, N,N'-bis(1-ethyl-3-methylpentyl)-p-phenylenediamine, N,N'-bis(1-methylheptyl)-p-phenylenediamine, N,N'-dicyclohexyl-p-phenylenediamine, N,N'-diphenyl-p-phenylenediamine, N,N'-bis(2-naphthyl)-p-phenylenediamine, N-isopropyl-N'-phenyl-p-phenylenediamine, N-(1,3-dimethylbutyl)-N'-phenyl-p-phenylenediamine, N-(1-methylheptyl)-N'-phenyl-p-phenylenediamine, N-cyclohexyl-N'-phenyl-p-phenylenediamine, 4-(p-toluenesulfamoyl)diphenylamine, N,N'-dimethyl-N,N'-di-sec-butyl-p-phenylenediamine, diphenylamine, N-allyldiphenylamine, 4-isopropoxydiphenylamine, N-phenyl-1-naphthylamine, N-(4-tert-octylphenyl)-1-naphthylamine, N-phenyl-2-naphthylamine, octylated diphenylamine, for example p,p'-di-tert-octyldiphenylamine, 4-n-butylaminophenol, 4-butyrylaminophenol, 4-nonanoylaminophenol, 4-dodecanoylaminophenol, 4-octadecanoylaminophenol, bis(4-methoxyphenyl)amine, 2,6-di-tert-butyl-4-dimethylamino-methylphenol, 2,4'-diaminodiphenylmethane, 4,4'-diaminodiphenylmethane, N,N,N',N'-tetramethyl-4,4'-diaminodiphenylmethane, 1,2-bis[(2-methylphenyl)amino]ethane, 1,2-bis(phenylamino)propane, (o-tolyl)biguanide, bis[4-(1',3'-dimethylbutyl)phenyl]amine, tert-octylated N-phenyl-1-naphthylamine, a mixture of mono- and dialkylated tert-butyl/tert-octyldiphenylamines, a mixture of mono- and dialkylated nonyldiphenylamines, a mixture of mono- and dialkylated dodecyldiphenylamines, a mixture of mono- and dialkylated isopropyl/isohexyldiphenylamines, a mixture of mono- and dialkylated tert-butyldiphenylamines, 2,3-dihydro-3,3-dimethyl-4H-1,4-benzothiazine, phenothiazine, a mixture of mono- and dialkylated tert-butyl/tert-octylphenothiazines, a mixture of mono- and dialkylated tert-octylphenothiazines, N-allylphenothiazine, dinonylphenothiazine, mono-nonylphenothiazine, a mixture of mono- and dialkylated nonylphenothiazine, N,N,N',N'-tetraphenyl-1,4-diaminobut-2-ene, a mixture of one of the above disclosed unsubstituted or substituted diphenylamine with one of the above disclosed unsubstitutued or substituted phenothiazine.
2. UV absorbers and light stabilizers
   2.1. 2-(2'-Hydroxyphenyl)benzotriazoles, for example 2-(2'-hydroxy-5'-methylphenyl)benzotriazole, 2-(3',5'-di-tert-butyl-2'-hydroxyphenyl)benzotriazole, 2-(5'-tert-butyl-2'-hydroxyphenyl)benzotriazole, 2-(2'-hydroxy-5'-(1,1,3,3-tetramethylbutyl)phenyl)benzotriazole, 2-(3',5'-di-tert-butyl-2'-hydroxyphenyl)-5-chlorobenzotriazole, 2-(3'-tert-butyl-2'-hydroxy-5'-methylphenyl)-5-chlorobenzotriazole, 2-(3'-sec-butyl-5'-tert-butyl-2'-hydroxyphenyl)benzobriazole, 2-(2'-hydroxy-4'-octyloxyphenyl)benzotriazole, 2-(3',5'-di-tert-amyl-2'-hydroxyphenyl)benzotriazole, 2-(3',5'-bis(α,α-dimethyl benzyl)-2'-hydroxyphenyl)benzotriazole, 2-(3'-tert-butyl-2'-hydroxy-5'-(2-octyloxycarbonylethyl)phenyl)-5-chlorobenzotriazole, 2-(3'-tert-butyl-5'-[2-(2-ethylhexyloxy)carbonylethyl]-2'-hydroxyphenyl)-5-chlorobenzotriazole, 2-(3'-tert-butyl-2'-hydroxy-5'-(2-methoxycarbonylethyl)phenyl)-5-chlorobenzotriazole, 2-(3'-tert-butyl-2'-hydroxy-5'-(2-methoxycarbonylethyl)phenyl)benzotriazole, 2-(3'-tert-butyl-2'-hydroxy-5'-(2-octyloxycarbonyl-ethyl)phenyl)benzotriazole, 2-(3'-tert-butyl-5'-[2-(2-ethylhexyloxy)carbonylethyl]-2'-hydroxyphenyl)benzotriazole, 2-(3'-dodecyl-2'-hydroxy-5'-methylphenyl)benzotriazole, 2-(3'-tert-butyl-2'-hydroxy-5'-(2-isooctyloxycarbonylethyl)phenylbenzotriazole, 2,2'-methylenebis[4-(1,1,3,3-tetramethylbutyl)-6-benzotriazole-2-ylphenol]; the transesterification product of 2-[3'-tert-butyl-5'-(2-methoxycarbonylethyl)-2'-hydroxyphenyl]-2H-benzotriazole with polyethylene glycol 300; [R-CH₂CH₂-COO-CH₂CH₂⁆₂, where R = 3'-tert-butyl-4'-hydroxy-5'-2H-benzotriazol-2-ylphenyl, 2-[2'-hydroxy-3'-(α,α-dimethylbenzyl)-5'-(1,1,3,3-tetramethylbutyl)phenyl]benzotriazole; 2-[2'-hydroxy-3'-(1,1,3,3-tetramethylbutyl)-5'-(α,α-dimethylbenzyl)phenyl]benzotriazole.
   2.2. 2-Hydroxybenzophenones and Formamidines, for example the 4-hydroxy, 4-methoxy, 4-octyloxy, 4-decyloxy, 4-dodecyloxy, 4-benzyloxy, 4,2',4'-trihydroxy and 2'-hydroxy-4,4'-dimethoxy benzophenones; N-alkyl-N,N'-diarylformamidines, for example, benzoic acid, 4-[[(methylphenylamino)methylene]amino] ethyl ester [Tinuvin 101 (RTM), Ciba Specialty Chemicals Inc.]; benzoic acid, 4-[[(ethylphenylamino)methylene]amino] ethyl ester; 2-propenoic acid, 3-(4-methoxyphenyl)-, 2-ethylhexyl ester [Uvinul 3088 (RTM), BASF]; 2-propenoic acid, 2-cyano-3,3-diphenyl-, ethyl ester [Uvinul 3035 (RTM), BASF]; or 2-propenoic acid, 2-cyano-3,3-diphenyl-, 2-ethylhexyl ester [Uvinul 3039 (RTM), BASF].
   2.3. Esters of substituted and unsubstituted benzoic acids, for example 4-tert-butylphenyl salicylate, phenyl salicylate, octylphenyl salicylate, dibenzoyl resorcinol, bis(4-tert-butylbenzoyl)resorcinol, benzoyl resorcinol, 2,4-di-tert-butylphenyl 3,5-di-tert-butyl-4-hydroxybenzoate, hexadecyl 3,5-di-tert-butyl-4-hydroxybenzoate, octadecyl 3,5-di-tert-butyl-4-hydroxybenzoate, 2-methyl-4,6-di-tert-butylphenyl 3,5-di-tert-butyl-4-hydroxybenzoate.
   2.4. Acrylates, for example ethyl α-cyano-β,β-diphenylacrylate, isooctyl α-cyano-β,β-diphenylacrylate, methyl α-carbomethoxycinnamate, methyl α-cyano-β-methyl-p-methoxycinnamate, butyl α-cyano-β-methyl-p-methoxycinnamate, methyl α-carbomethoxy-p-methoxycinnamate and N-(β-carbomethoxy-β-cyanovinyl)-2-methylindoline.
   2.5. Nickel compounds, for example nickel complexes of 2,2'-thiobis[4-(1,1,3,3-tetramethylbutyl)phenol], such as the 1:1 or 1:2 complex, with or without additional ligands such as n-butylamine, triethanolamine or N-cyclohexyldiethanolamine, nickel dibutyldithiocarbamate, nickel salts of the monoalkyl esters, e.g. the methyl or ethyl ester, of 4-hydroxy-3,5-di-tert-butylbenzylphosphonic acid, nickel complexes of ketoximes, e.g. of 2-hydroxy-4-methylphenylundecylketoxime, nickel complexes of 1-phenyl-4-lauroyl-5-hydroxypyrazole, with or without additional ligands.
   2.6. Sterically hindered amines, for example bis(2,2,6,6-tetramethyl-4-piperidyl)sebacate, bis(2,2,6,6-tetramethyl-4-piperidyl)succinate, bis(1,2,2,6,6-pentamethyl-4-piperidyl)sebacate, bis(1-octyloxy-2,2,6,6-tetramethyl-4-piperidyl)sebacate, bis(1,2,2,6,6-pentamethyl-4-piperidyl) n-butyl-3,5-di-tert-butyl-4-hydroxybenzylmalonate, the condensate of 1-(2-hydroxyethyl)-2,2,6,6-tetramethyl-4-hydroxypiperidine and succinic acid, linear or cyclic condensates of N,N'-bis(2,2,6,6-tetramethyl-4-piperidyl)hexamethylenediamine and 4-tert-octylamino-2,6-dichloro-1,3,5-triazine, tris(2,2,6,6-tetramethyl-4-piperidyl)nitrilotriacetate, tetrakis(2,2,6,6-tetramethyl-4-piperidyl)-1,2,3,4-butanetetracarboxylate, 1,1'-(1,2-ethanediyl)-bis(3,3,5,5-tetramethylpiperazinone), 4-benzoyl-2,2,6,6-tetramethylpiperidine, 4-stearyloxy-2,2,6,6-tetramethylpiperidine, bis(1,2,2,6,6-pentamethylpiperidyl)-2-n-butyl-2-(2-hydroxy-3,5-di-tert-butylbenzyl)malonate, 3-n-octyl-7,7,9,9-tetramethyl-1,3,8-triazaspiro[4.5]decane-2,4-dione, bis(1-octyloxy-2,2,6,6-tetramethylpiperidyl)sebacate, bis(1-octyloxy-2,2,6,6-tetramethylpiperidyl)succinate, linear or cyclic condensates of N,N'-bis(2,2,6,6-tetramethyl-4-piperidyl)hexamethylenediamine and 4-morpholino-2,6-dichloro-1,3,5-triazine, the condensate of 2-chloro-4,6-bis(4-n-butylamino-2,2,6,6-tetramethylpiperidyl)-1,3,5-triazine and 1,2-bis(3-aminopropylamino)ethane, the condensate of 2-chloro-4,6-di-(4-n-butylamino-1,2,2,6,6-pentamethylpiperidyl)-1,3,5-triazine and 1,2-bis(3-aminopropylamino)ethane, 8-acetyl-3-dodecyl-7,7,9,9-tetramethyl-1,3,8-triazaspiro[4.5]decane-2,4-dione, 3-dodecyl-1-(2,2,6,6-tetramethyl-4-piperidyl)pyrrolidine-2,5-dione, 3-dodecyl-1-(1,2,2,6,6-pentamethyl-4-piperidyl)pyrrolidine-2,5-dione, a mixture of 4-hexadecyloxy- and 4-stearyloxy-2,2,6,6-tetramethylpiperidine, a condensate of N,N'-bis(2,2,6,6-tetramethyl-4-piperidyl)hexamethylenediamine and 4-cyclohexylamino-2,6-dichloro-1,3,5-triazine, a condensate of 1,2-bis(3-aminopropylamino)ethane and 2,4,6-tri-chloro-1,3,5-triazine as well as 4-butylamino-2,2,6,6-tetramethylpiperidine (CAS Reg. No. [136504-96-6]); a condensate of 1,6-hexanediamine and 2,4,6-trichloro-1,3,5-triazine as well as N,N-dibutylamine and 4-butylamino-2,2,6,6-tetramethylpiperidine (CAS Reg. No. [192268-64-7]); N-(2,2,6,6-tetramethyl-4-piperidyl)-n-dodecylsuccinimide, N-(1,2,2,6,6-pentamethyl-4-piperidyl)-n-dodecylsuccinimide, 2-undecyl-7,7,9,9-tetramethyl-1-oxa-3,8-diaza-4-oxo-spiro[4,5]decane, a reaction product of 7,7,9,9-tetramethyl-2-cycloundecyl-1-oxa-3,8-diaza-4-oxospiro-[4,5]decane and epichlorohydrin, 1,1-bis(1,2,2,6,6-pentamethyl-4-piperidyloxycarbonyl)-2-(4-methoxyphenyl)ethene, N,N'-bis-formyl-N,N'-bis(2,2,6,6-tetramethyl-4-piperidyl)hexamethylenediamine, a diester of 4-methoxymethylenemalonic acid with 1,2,2,6,6-pentamethyl-4-hydroxypiperidine, poly[methylpropyl-3-oxy-4-(2,2,6,6-tetramethyl-4-piperidyl)]siloxane, a reaction product of maleic acid anhydride-α-olefin copolymer with 2,2,6,6-tetramethyl-4-aminopiperidine or 1,2,2,6,6-pentamethyl-4-aminopiperidine.
   2.7. Oxamides, for example 4,4'-dioctyloxyoxanilide, 2,2'-diethoxyoxanilide, 2,2'-dioctyloxy5,5'-di-tert-butoxanilide, 2,2'-didodecyloxy-5,5'-di-tert-butoxanilide, 2-ethoxy-2'-ethyloxanilide, N,N'-bis(3-dimethylaminopropyl)oxamide, 2-ethoxy-5-tert-butyl-2'-ethoxanilide and its mixture with 2-ethoxy-2'-ethyl-5,4'-di-tert-butoxanilide, mixtures of o- and p-methoxydisubstituted oxanilides and mixtures of o- and p-ethoxy-disubstituted oxanilides.
   2.8. 2-(2-Hydroxyphenyl)-1,3,5-triazines, for example 2,4,6-tris(2-hydroxy-4-octyloxyphenyl)-1,3,5-triazine, 2-(2-hydroxy-4-octyloxyphenyl)-4,6-bis(2,4-dimethylphenyl)-1,3,5-triazine, 2-(2,4-dihydroxyphenyl)-4,6-bis(2,4-dimethylphenyl)-1,3,5-triazine, 2,4-bis(2-hydroxy-4-propyl-oxyphenyl)-6-(2,4-dimethylphenyl)-1,3,5-triazine, 2-(2-hydroxy-4-octyloxyphenyl)-4,6-bis(4-methylphenyl)-1,3,5-triazine, 2-(2-hydroxy-4-dodecyloxyphenyl)-4,6-bis(2,4-dimethylphenyl)-1,3,5-triazine, 2-(2-hydroxy-4-tridecyloxyphenyl)-4,6-bis(2,4-dimethylphenyl)-1,3,5-triazine, 2-[2-hydroxy-4-(2-hydroxy-3-butyloxypropoxy)phenyl]-4,6-bis(2,4-dimethyl)-1,3,5-triazine, 2-[2-hydroxy-4-(2-hydroxy-3-octyloxypropyloxy)phenyl]-4,6-bis(2,4-dimethyl)-1,3,5-triazine, 2-[4-(dodecyloxy/tridecyloxy-2-hydroxypropoxy)-2-hydroxyphenyl]-4,6-bis(2,4-dimethylphenyl)-1,3,5-triazine, 2-[2-hydroxy-4-(2-hydroxy-3-dodecyloxypropoxy)phenyl]-4,6-bis(2,4-dimethyl-phenyl)-1,3,5-triazine, 2-(2-hydroxy-4-hexyloxy)phenyl-4,6-diphenyl-1,3,5-triazine, 2-(2-hydroxy-4-methoxyphenyl)-4,6-diphenyl-1,3,5-triazine, 2,4,6-tris[2-hydroxy-4-(3-butoxy-2-hydroxypropoxy)phenyl]-1,3,5-triazine, 2-(2-hydroxyphenyl)-4-(4-methoxyphenyl)-6-phenyl-1,3,5-triazine, 2-{2-hydroxy-4-[3-(2-ethylhexyl-1-oxy)-2-hydroxypropyloxy]phenyl}-4,6-bis-(2,4-dimethylphenyl)-1,3,5-triazine.
3. Metal deactivators, for example N,N'-diphenyloxamide, N-salicylal-N'-salicyloyl hydrazine, N,N'-bis(salicyloyl)hydrazine, N,N'-bis(3,5-di-tert-butyl-4-hydroxyphenylpropionyl)hydrazine, 3-salicyloylamino-1,2,4-triazole, bis(benzylidene)oxalyl dihydrazide, oxanilide, isophthaloyl dihydrazide, sebacoyl bisphenylhydrazide, N,N'-diacetyladipoyl dihydrazide, N,N'-bis(salicyloyl)oxalyl dihydrazide, N,N'-bis(salicyloyl)thiopropionyl dihydrazide.
4. Phosphites and phosphonites, for example triphenyl phosphite, diphenylalkyl phosphites, phenyldialkyl phosphites, tris(nonylphenyl) phosphite, trilauryl phosphite, trioctadecyl phosphite, distearylpentaerythritol diphosphite, tris(2,4-di-tert-butylphenyl) phosphite, diisodecyl pentaerythritol diphosphite, bis(2,4-di-tert-butylphenyl)pentaerythritol diphosphite, bis(2,4-dicumylphenyl)pentaerythritol diphosphite, bis(2,6-di-tert-butyl-4-methylphenyl)pentaerythritol diphosphite, diisodecyloxypentaerythritol diphosphite, bis(2,4-di-tert-butyl-6-methylphenyl)-pentaerythritol diphosphite, bis(2,4,6-tris(tert-butylphenyl)pentaerythritol diphosphite, tristearyl sorbitol triphosphite, tetrakis(2,4-di-tert-butylphenyl) 4,4'-biphenylene diphosphonite, 6-isooctyloxy-2,4,8,10-tetra-tert-butyl-12H-dibenz[d,g]-1,3,2-dioxaphosphocin, bis(2,4-di-tert-butyl-6-methylphenyl)methyl phosphite, bis(2,4-di-tert-butyl-6-methylphenyl)ethyl phosphite, 6-fluoro-2,4,8,10-tetra-tert-butyl-12-methyl-dibenz[d,g]-1,3,2-dioxaphosphocin, 2,2',2"-nitrilo-[triethyltris(3,3',5,5'-tetra-tert-butyl-1,1'-biphenyl-2,2'-diyl)phosphite], 2-ethylhexyl(3,3',5,5'-tetra-tert-butyl-1,1'-biphenyl-2,2'-diyl)phosphite, 5-butyl-5-ethyl-2-(2,4,6-tri-tert-butylphenoxy)-1,3,2-dioxaphosphirane.
5. Hydroxylamines, for example N,N-dibenzylhydroxylamine, N,N-diethylhydroxylamine, N,N-dioctylhydroxylamine, N,N-dilaurylhydroxylamine, N,N-ditetradecylhydroxylamine, N,N-dihexadecylhydroxylamine, N,N-dioctadecylhydroxylamine, N-hexadecyl-N-octadecylhydroxylamine, N-heptadecyl-N-octadecylhydroxylamine, N,N-dialkylhydroxylamine derived from hydrogenated tallow amine.
6. Nitrones, for example N-benzyl-alpha-phenylnitrone, N-ethyl-alpha-methylnitrone, N-octyl-alpha-heptylnitrone, N-lauryl-alpha-undecylnitrone, N-tetradecyl-alpha-tridecylnitrone, N-hexadecyl-alpha-pentadecylnitrone, N-octadecyl-alpha-heptadecylnitrone, N-hexadecyl-alpha-heptadecylnitrone, N-ocatadecyl-alpha-pentadecylnitrone, N-heptadecyl-alpha-hepta-decylnitrone, N-octadecyl-alpha-hexadecylnitrone, nitrone derived from N,N-dialkylhydroxylamine derived from hydrogenated tallow amine.
7. Thiosynergists, for example dilauryl thiodipropionate or distearyl thiodipropionate.
8. Peroxide scavengers, for example esters of β-thiodipropionic acid, for example the lauryl, stearyl, myristyl or tridecyl esters, mercaptobenzimidazole or the zinc salt of 2-mercapto-benzimidazole, zinc dibutyldithiocarbamate, dioctadecyl disulfide, pentaerythritol tetrakis(p-dodecylmercapto)propionate.
9. Basic co-stabilisers, for example melamine, polyvinylpyrrolidone, dicyandiamide, triallyl cyanurate, urea derivatives, hydrazine derivatives, amines, polyamides, polyurethanes, alkali metal salts and alkaline earth metal salts of higher fatty acids, for example calcium stearate, zinc stearate, magnesium behenate, magnesium stearate, sodium ricinoleate and potassium palmitate, antimony pyrocatecholate or zinc pyrocatecholate.
10. Fillers and reinforcing agents, for example calcium carbonate, silicates, glass fibres, glass bulbs, asbestos, talc, kaolin, mica, barium sulfate, metal oxides and hydroxides, carbon black, graphite, wood flour and flours or fibers of other natural products, synthetic fibers.
11. Other additives, for example plasticisers, lubricants, emulsifiers, pigments, rheology additives, catalysts, flow-control agents, optical brighteners, flameproofing agents, antistatic agents and blowing agents.
12. Benzofuranones and indolinones, for example those disclosed in U.S. 4,325,863; U.S.
4,338,244; U.S. 5,175,312; U.S. 5,216,052; U.S. 5,252,643; DE-A-4316611; DE-A-4316622; DE-A-4316876; EP-A-0589839; EP-A-0591102 or EP-A-1291384 or 3-[4(2-acetoxyethoxy)phenyl]-5,7-di-tert-butylbenzofuran-2-one, 5,7-di-tert-butyl-3-[4-(2-stearoyloxyethoxy)phenyl]benzofuran-2-one, 3,3'-bis[5,7-di-tert-butyl-3-(4-[2-hydroxyethoxy]phenyl)benzofuran-2-one], 5,7-di-tert-butyl-3-(4-ethoxyphenyl)benzofuran-2-one, 3-(4-acetoxy-3,5-dimethylphenyl)-5,7-di-tert-butylbenzofuran-2-one, 3-(3,5-dimethyl-4-pivaloyloxyphenyl)-5,7-di-tert-butylbenzofuran-2-one, 3-(3,4-dimethylphenyl)-5,7-di-tert-butylbenzofuran-2-one, 3-(2,3-dimethylphenyl)-5,7-di-tert-butylbenzofuran-2-one or 3-(2-acetyl-5-isooctylphenyl)-5-isooctylbenzofuran-2-one.

The costabilizers are added, for example, in concentration of 0.01 to 10%, relative to the total weight of the synthetic polymer to be stabilized.

Preferred further additives are light-stabilizers and/or aminic antioxidants. Preferred aminic antioxidants are for example disclosed in item 1.19. Processing stabilizers according to the invention are benzofuranones (item 12), phosphites or phosphonites (item 4).

Preferably, as aminic antioxidants a mixture of unsubstituted or substituted diphenylamines and of unsubstituted or substituted phenothiazines is used.

The presentapplication also discloses new compounds of the formula Ia, Ib, Ic, Id and Ie.

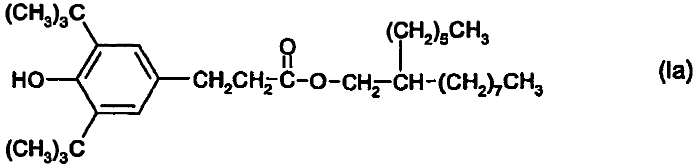

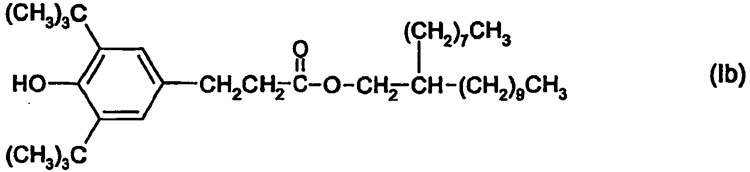

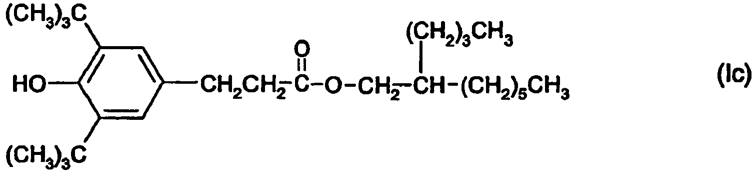

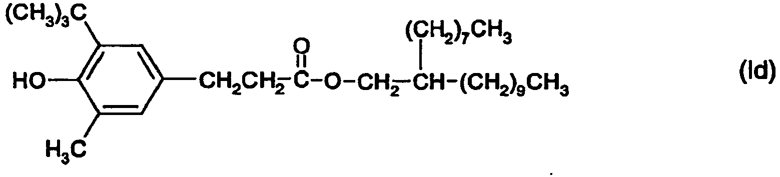

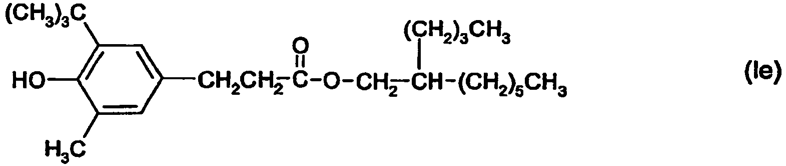

The presentapplication also discloses a process for stabilizing a polyether polyol, a polyester polyol or a polyurethane against oxidative, thermal or light-induced degradation and reducing the fogging contribution of the polymers, which comprises incorporating therein or applying thereto at least a liquid compound of the formula I.

Thus, the present invention relates to a process for stabilizing a polyether polyol, a polyester polyol or a polyurethane against oxidative, thermal or light-induced degradation and reducing the fogging contribution of the polymers, which comprises incorporating therein or applying thereto at least a liquid compound of the formula I and benzofuranones, phosphites, phosphonites or phenothiazines as processing stabilizers.

Another preferred subject of the present application is therefore the use of the liquid compounds of the formula I as stabilizers for polyether polyols, polyester polyols or polyurethanes against oxidative, thermal or light-induced degradation and as reducers of fogging contribution of the polymers.

Thus, a preferred subject of the present invention is the use of at least a liquid compound of the formula I and of benzofuranones, phosphites, phosphonites or phenothiazines as processing stabilizers for stabilizing a polyether polyol, a polyester polyol or polyurethane against oxidative, thermal or light-induced degradation and for reducing of fogging contribution of the polymers.

The examples which follow illustrate the invention in more detail. Parts and percentages are by weight.

### Example 1: Preparation of compound 101 of the formula Ia.

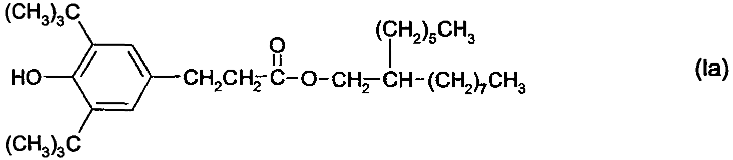

58.48 g (0.20 mol) of methyl-3-(3',5'-di-t-butyl-4'hydroxyphenyl)-propionate (Ciba Specialty Chemicals Inc.) is melted in 50.59 g (0.2087 mol) of 2-hexyl-1-decanol (Isofol 16, purity: 97 %, from Condea) by heating in a percolating stream of nitrogen in a 3-necked flask equipped with a stirrer, a thermometer and a short reflux-condenser. Then a catalytic amount of 0.80 g (0.0032 mol) of dibutyl-tin-oxide (Fluka, purum) is added with stirring and the reaction mixture is heated up to a bath-temperature of 190°C. A clear, slightly yellow solution results which is kept with stirring at that temperature for further 16 hours. The evolving methanol is blown off by a vivid nitrogen stream. Then the reaction mass is cooled to room temperature and the liquid material is filtered through a small amount of silicagel 60 (Merck) to remove the catalyst. The residue yields 100.50 g (100 % of theory) of the compound 101 of the formula Ia. Analysis for C₃₃H₅₈O₃: calculated: C 78.83; H 11.63%. found: C 78.55; H 11.48%.

### Example 2: Preparation of the compound 102 of the formula Ib.

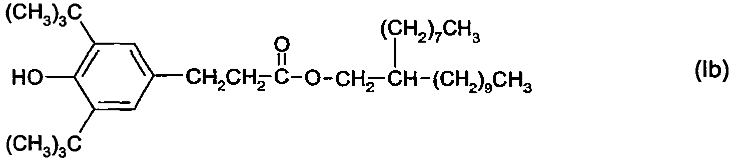

58.48 g (0.20 mol) of methyl-3-(3',5'-di-t-butyl-4'hydroxyphenyl)-propionate (Ciba Specialty Chemicals Inc.) is melted in 59.70 g (0.209 mol) of 2-octyl-1-dodecanol (Isofol 20, from Condea) by heating in a percolating stream of nitrogen in a 3-necked flask equipped with a stirrer, a thermometer and a short reflux-condenser. Then a catalytic amount of 0.80 g (0.0032 mol) of dibutyl-tin-oxide (Fluka, purum) is added with stirring and the reaction mixture heated up to a bath-temperature of 190°C. A clear, slightly yellow solution results which is kept with stirring at that temperature for further 16 hours. The evolving methanol is blown off by a vivid nitrogen stream. Then the reaction mass is cooled to room temperature and the liquid material is filtered through a small amount of silicagel 60 (Merck) to remove the catalyst. The residue yields 110.98 g (99 % of theory) of the compound 102 of the formula Ib. Analysis for C₃₇H₆₆O₃: calculated: C 79.51; H 11.90%. found: C 79.36; H 11.96%.

### Example 3: Preparation of the compound 103 of the formula Ic.

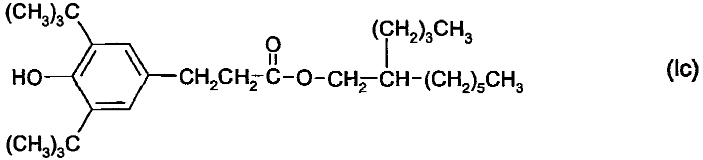

1.194 kg (7.619 mol) of methyl-3-(3',5'-di-t-butyl-4'hydroxyphenyl)-propionate (Ciba Specialty Chemicals Inc.) is melted in 1.441 kg (7.75 mol) of 2-butyl-1-octanol (Isofol 12, from Condea) by heating in a percolating stream of nitrogen in a 3-necked flask equipped with a stirrer, a thermometer and a short reflux-condenser. Then a catalytic amount of 22.52 g (0.091 mol) of aluminum-tris-butanolate (95% ,Fluka pract.) is added with stirring and the reaction mixture heated up to a bath-temperature of 170°C. A clear, slightly yellow solution results which is kept with stirring at that temperature for further 16 hours. The evolving methanol is blown off by a vivid nitrogen stream. Then the reaction mass is cooled to room temperature and the liquid material is filtered through a small amount of silicagel 60 (Merck) to remove the catalyst. The residue yields 3.11 kg of the compound 103 of the formula Ic. TLC (Merck TLC-plates, silicagel 60 F 254, solvent: hexane/acetone = 6/1). Main spot at Rf = 0.45.

### Example 4: Preparation of compound 104 of the formula Id.

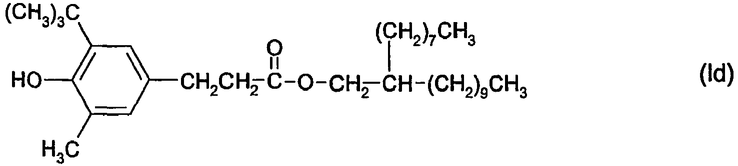

500.68 g (2 mol) of methyl-3-(3'-t-butyl-5'-methyl-4'-hydroxyphenyl)-propionate (Ciba Specialty Chemicals Inc.) is melted in 605.84 (2.033 mol) of 2-octyl-1-dodecanol (Isofol 20 from Condea) by heating under nitrogen in a 3-necked flask, equipped with a stirrer, a thermometer, a reflux-condenser and a trap cooled to -78° C, on top of the condenser. Then a catalytic amount of 6.0 g (0.024 moles) aluminum-tris-butanolate (95 %, Fluka pract.) is added to the reaction mixture. The trap is hooked to a partial vacuum of 200 hPa. Within 60 minutes the temperature is raised to 180°C. After further three hours the partial vacuum is changed to 35 hPa. After 10 hours a total amount of 60.90 g (theoretical: 64 g) methanol is distilled off and is collected in the cooling trap. The reaction mixture is slightly yellow and is cooled down from 180° C to 80° C. Then 350 ml of deionized water is added and stirring is continued. The organic layer is separated and filtered with suction through diatomaceous earth (Hyflo). The filter is rinsed with 200 ml of hexane, which is combined with the filtered organic part. The combined organic layers are washed twice with 350 ml of deionized water. The water phases are carefully separated whereby an end-pH of 5 - 6 is reached during the last washing-operation. The organic material is again filtered with suction through Hyflo. Then the hexane is distilled off at 80° C in a partial vacuum of 200 hPa. The resulting liquid is dried with stirring at 125°C/25 - 30 hPa during a further hour. 1016.6 g (98 % of theory) of the compound 104 of the formula Id is obtained. Analysis (C₃₄H₆₀O₃): calculated: C 79.01; H 11.70 %. found: C 79.12; H 11.62 %.

### Example 5: Preparation of polyether/polyurethane soft foams as well as the stabilization thereof.

0.72 g (0.45 %, based on the polyol) of a stabilizer according to Table 1 is dissolved in 160 g of a polyether polyol [Petol 46-3MB (RTM) (trifunctional polyether polyol having primary hydroxyl groups; hydroxyl number 48 mg KOH/g, water content less than 0.1 %, acid number less than 0.1 mg KOH/g)], 8.16 g of a solution consisting of 1.6 g Tecostab (RTM) BF 2370 (supplied by Goldschmidt, Germany), 0.16 g Tegoamin ZE1 (supplied by Goldschmidt, Germany) and 6.4 g of deionized water are added and the reaction mixture is stirred vigorously for 10 seconds at 2600 rpm. 0.67 g Kosmos EF (supplied by Goldschmidt, Germany) is then added and the reaction mixture is again stirred vigorously for 18 seconds at 2600 rpm. 80.96 g of an isocyanate [Lupranat T80 (RTM), supplied by BASF; toluylene-2,4- and toluylene-2,6-diisocyanate mixture] is then added with continuous stirring for 5 to 7 seconds at 2600 rpm. The mixture is then poured into a 20 x 20 x 20 cm cake-box and the exothermic temperature is measured during foaming to a foam block. The foam blocks are cooled and stored at room temperature for 24 hours. The next day the foams are cut into thin tubes (2cm thick, 1.5 cm in diameter).

Dynamic heat aging of foam samples is used as a measure of scorch resistance (Dynamic Alu Block Test). The foam samples are typically heated in an oven or an aluminum block and scorch resistance is assessed by measuring the color change. In the "dynamic" heat aging test the temperature is increased at a constant rate and the color change determined as a function of the temperature (30 minutes at temperatures between 170 and 230 °C). The foam color quality is reported in terms of Yellowness Index (YI) determined on the foam samples in accordance with the ASTM 1926-70 Yellowness Test. Low YI values denote little discoloration, high YI values severe discoloration of the samples. The whiter the foam the better is the foam stabilized.

Substances that can be released from the foam are reported in terms of volatile organic compounds (VOC) or gaseous emissions and condensable emissions (FOG) determined on the foam samples in accordance with the method PB VWL 709 ("Determination of gaseous (VOC) and by thermodesorption", developed by Daimler Chrysler Ltd). For this purpose, a certain amount of material is heated in an inert gas flow, and thereby released substances are then frozen in the deep-freeze injector of a gas chromatograph. After separating the mixture, the individual substances are identified by a mass selective detector, as far as possible. The VOC and FOG measurements are done with the same sub-part of the material sample. The quantification of the gaseous emissions (VOC) is done using an external toluene standard, the quantification of the condensable emissions (FOG) by using hexadecane (C₁₆-n-alkane). Reported are the concentrations in ppm (mg/kg), as total emissions, in toluene- or hexadecane- equivalents. Low VOC values denote little gaseous emissions; high VOC values denote severe gaseous emissions of the foam samples. Low FOG values denote little condensable emissions; high FOG values denote severe condensable emissions of the foam samples. The lower the VOC/FOG values the better is the foam in terms of releasable emissions. The results are summarized in Table 1.

**Table 1:**

| Example | Stabilizer | VOC (mg/kg) | FOG (mg/kg) |
|---|---|---|---|
| 3a^{a)} | 0.35 % IRGANOX 1135 ^{c)} | 25 | 360 |
| | 0.05 % IRGAFOS 38 ^{d)} | | |
| | 0.05 % PS-1 ^{e)} | | |
| 3b^{b)} | 0.35 % Compound 101 ^{f)} | 25 | 40 |
| | 0.05 % IRGAFOS 38 ^{d)} | | |
| | 0.05 % PS-1 ^{e)} | | |
| 3c^{b)} | 0.35 % Compound 102 ^{g)} | 25 | 35 |
| | 0.05 % IRGAFOS 38 ^{d)} | | |
| | 0.05 % PS-1 ^{e)} | | |

| | | | |
|---|---|---|---|
| a) Comparison example. b) Example according to the invention. c) Irganox 1135 (RTM) (Ciba Specialty Chemicals Inc.) is a phenolic antioxidant of the formula AO-1. 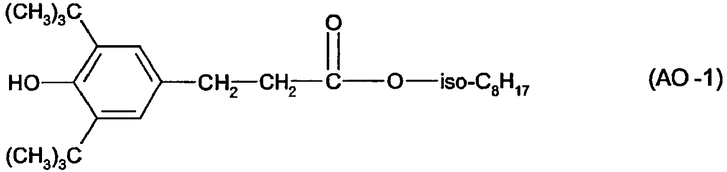 d) Irgafos 38 (RTM) is bis(2,4-di-tert-butyl-6-methylphenyl)ethyl phosphite. e) PS-1 is 3-(2-actyl-5-isooctylphenyl)-5-isooctylbenzofuran-2-one of the formula B1. 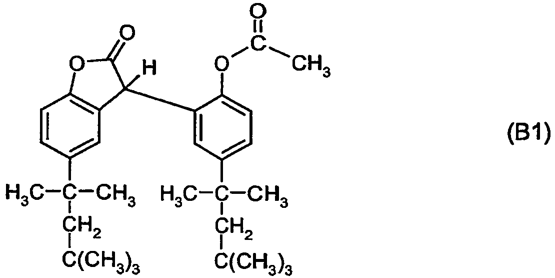 f) The preparation of compound 101 is disclosed in Example 1. g) The preparation of compound 102 is disclosed in Example 2. | | | |

## Claims

1. A composition comprising
a) a polyether polyol, a polyester polyol or a polyurethane susceptible to oxidative, thermal or light-induced degradation; and
b) at least a liquid compound of the formula I 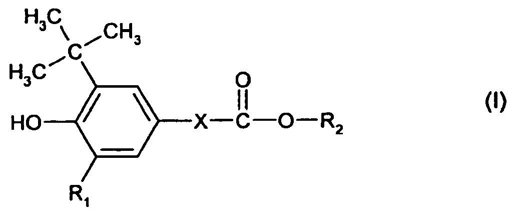 wherein
R₁ is C₁-C₄alkyl,
R₂ is a branched C₁₂-C₂₅alkyl, and
X is C₁-C₈alkylene or C₁-C₄alkyl substituted C₂-C₈alkylene; and
c) as processing stabilizers benzofuranones, phosphites, phosphonites or phenothiazines.

2. A composition according to claim 1, wherein
R₁ is methyl or tert-butyl,
R₂ is a branched C₁₄-C₂₅alkyl, and
X is C₁-C₄alkylene or a methyl substituted C₂-C₄alkylene.

3. A composition according to claim 1, wherein X is ethylene.

4. A composition according to claim 1, wherein R₂ is a branched C₁₅-C₂₂alkyl.

5. A composition according to claim 1, wherein
R₁ is methyl or tert-butyl,
R₂ is a branched C₁₆-C₂₀alkyl, and
X is ethylene.

6. A composition according to claim 1, wherein component (b) is present in an amount of 0.01 to 10 % based on the weight of component (a).

7. A process for stabilizing a polyether polyol, a polyester polyol or a polyurethane against oxidative, thermal or light-induced degradation and reducing the fogging contribution of the polymers, which comprises incorporating therein or applying thereto at least a liquid compound of the formula I and processing stabilizers according to claim 1.

8. Use of at least a liquid compound of the formula I and processing stabilizers according to claim 1 for stabilizing a polyether polyol, a polyester polyol or polyurethane against oxidative, thermal or light-induced degradation and for reducing of fogging contribution of the polymers.

## Patentansprüche

1. Zusammensetzung, umfassend
a) ein Polyether-polyol, ein Polyester-polyol oder ein Polyurethan, anfällig für oxidativen, thermischen oder durch Licht induzierten Abbau; und
b) mindestens eine flüssige Verbindung der Formel I 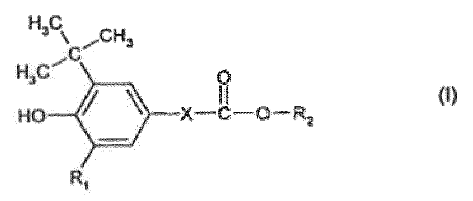 worin
R₁ C₁-C₄-Alkyl darstellt,
R₂ ein verzweigtes C₁₂-C₂₅-Alkyl darstellt, und
X C₁-C₈-Alkylen oder C₁-C₄-Alkyl substituiertes C₂-C₈-Alkylen darstellt; und
c) als Verarbeitungs-Stabilisatoren Benzofuranone, Phosphite, Phosphonite oder Phenothiazine.

2. Zusammensetzung nach Anspruch 1, wobei
R₁ Methyl oder tert-Butyl darstellt,
R₂ ein verzweigtes C₁₄-C₂₅-Alkyl darstellt, und
X C₁-C₄-Alkylen oder Methyl substituiertes C₂-C₄-Alkylen darstellt.

3. Zusammensetzung nach Anspruch 1, wobei X Ethylen darstellt.

4. Zusammensetzung nach Anspruch 1, wobei R₂ ein verzweigtes C₁₅-C₂₂-Alkyl darstellt.

5. Zusammensetzung nach Anspruch 1, wobei
R₁ Methyl oder tert-Butyl darstellt,
R₂ ein verzweigtes C₁₆-C₂₀-Alkyl darstellt, und
X Ethylen darstellt.

6. Zusammensetzung nach Anspruch 1, wobei Komponente (b) in einer Menge von 0,01 bis 10 %, basierend auf dem Gewicht von Komponente (a), vorliegt.

7. Verfahren zum Stabilisieren eines Polyether-polyols, eines Polyester-polyols oder eines Polyurethans gegen oxidativen, thermischen oder durch Licht induzierten Abbau und Vermindern des Fogging-Beitrags der Polymere, welches Einarbeiten darin oder Auftragen darauf von mindestens einer flüssigen Verbindung der Formel I und Verarbeitungs-Stabilisatoren nach Anspruch 1 umfasst.

8. Verwendung von mindestens einer flüssigen Verbindung der Formel I und Verarbeitungs-Stabilisatoren nach Anspruch 1 zum Stabilisieren eines Polyether-polyols, eines Polyester-polyols oder eines Polyurethans gegen oxidativen, thermischen oder durch Licht induzierten Abbau und zum Vermindern des Fogging-Beitrags der Polymere.

## Revendications

1. Composition comprenant
a) un polyol de polyéther, un polyol de polyester ou un polyuréthane sujet à la dégradation par oxydation, thermique ou provoquée par la lumière ; et
b) au moins un composé liquide représenté par la formule I 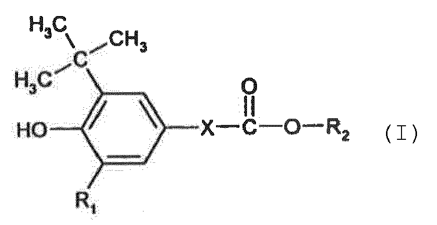 dans laquelle
R₁ est un groupe alkyle en C₁-C₄,
R₂ est un groupe alkyle en C₁₂-C₂₅ ramifié et
X est un groupe alkylène en C₁-C₈ ou alkylène en C₂-C₈ substitué par alkyle en C₁-C₄ ; et
c) en tant que stabilisants de traitement des benzofuranones, des phosphites, des phosphonites ou des phénothiazines.

2. Composition selon la revendication 1, dans laquelle
R₁ est un groupe méthyle ou *tert*-butyle,
R₂ est un groupe alkyle en C₁₄-C₂₅ ramifié et
X est un groupe alkylène en C₁-C₄ ou alkylène en C₂-C₄ substitué par méthyle.

3. Composition selon la revendication 1, dans laquelle X est un groupe éthylène.

4. Composition selon la revendication 1, dans laquelle R₂ est un groupe alkyle en C₁₅-C₂₂ ramifié.

5. Composition selon la revendication 1, dans laquelle
R₁ est un groupe méthyle ou *tert*-butyle,
R₂ est un groupe alkyle en C₁₆-C₂₀ ramifié et
X est un groupe éthylène.

6. Composition selon la revendication 1, dans laquelle le composant (b) est présent en une quantité de 0,01 à 10 % sur la base du poids du composant (a).

7. Procédé pour la stabilisation d'un polyol de polyéther, d'un polyol de polyester ou d'un polyuréthane contre une dégradation par oxydation, thermique ou provoquée par la lumière et la réduction de la contribution des polymères à la formation d'un voile, qui comprend l'incorporation dans ceux-ci ou l'application à ceux-ci d'au moins un composé liquide représenté par la formule I et de stabilisants de traitement selon la revendication 1.

8. Utilisation d'au moins un composé liquide représenté par la formule I et de stabilisants de traitement selon la revendication 1 pour la stabilisation d'un polyol de polyéther, d'un polyol de polyester ou d'un polyuréthane contre une dégradation par oxydation, thermique ou provoquée par la lumière et pour la réduction de la contribution des polymères à la formation d'un voile.
